(19) Europäisches Patentamt
European Patent Office
Office européen des brevets

(11) Publication number: 0 114 786
B1

(12) **EUROPEAN PATENT SPECIFICATION**

(45) Date of publication of patent specification: **24.09.86**

(21) Application number: **84810027.7**

(22) Date of filing: **16.01.84**

(51) Int. Cl.$^4$: **C 07 C 147/14,**
C 07 C 147/04, C 07 C 149/16,
C 08 F 28/02, C 08 J 9/42,
D 21 H 3/38 // C07C149/18,
C07C147/02

(54) **Perfluoralkylalkyl vinyl sulfoxides and sulfones.**

(30) Priority: **21.01.83 US 459739**

(43) Date of publication of application:
**01.08.84 Bulletin 84/31**

(45) Publication of the grant of the patent:
**24.09.86 Bulletin 86/39**

(84) Designated Contracting States:
**BE CH DE FR GB IT LI**

(56) References cited:
**US-A-3 578 717**

(73) Proprietor: **CIBA-GEIGY AG**
**Klybeckstrasse 141**
**CH-4002 Basel (CH)**

(72) Inventor: **Brace, Neal O.**
**1022 East North Path Avenue**
**Wheaton Illinois 60187 (US)**

**EP 0114 786 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to perfluoroalkylalkyl vinyl sulfoxides and sulfones.

Intermediates thereof, methods of preparation, and methods of use thereof to render cellulosic, and natural and synthetic polyamide, materials hydrophobic and oleophobic.

Accordingly, it is an object of the present invention to provide novel perfluoroalkylalkylene vinyl sulfoxides and sulfones — as well as telomers, polymers and co-polymers thereof — of the formula

$$R_f\!\left(CH_2-\underset{\underset{\displaystyle R_1}{|}}{CH}\right)_m S(O)_n-CH=CH_2 \qquad (1)$$

wherein $R_f$ is perfluoroalkyl of 3 to 18 carbon atoms, $R_1$ is hydrogen or lower alkyl, m is 1 to 3 and n is 1 or 2.

It is a further object of the present invention to provide a process for the manufacture of such perfluoroalkyl-alkyl vinyl sulfoxides and sulfones, as well as valuable intermediates in the preparation thereof.

It is yet a further object of the present invention to provide compositions and a process of rendering hydrophobic and oleophobic cellulosic, and natural and synthetic polyamide, materials using such compositions containing the perfluoroalkyl-alkyl vinyl sulfoxides and sulfones, or containing telomers, polymers or co-polymers thereof.

$R_f$ in the compounds of the formula (1) denotes a perfluoroalkyl radical preferably having 4 to 16 carbon atoms such as the perfluorinated derivatives of butyl, hexyl, heptyl, octyl, nonyl, decyl, dodecyl, tetradecyl and hexadecyl as well as isomers thereof. More preferred, $R_f$ is perfluoroalkyl of 4 to 16 carbon atoms. Also a mixture of $R_f$ groups as defined above may be present. Most preferred mixtures are those wherein the $R_f$ group has a range between about 4 and 12 carbon atoms with an average chain length between about 6 and 10 carbon atoms.

$R_1$ denotes hydrogen or lower alkyl, i.e. alkyl radicals having 1 to 5 carbon atoms such as methyl, ethyl, propyl, butyl and pentyl as well as isomers thereof. $R_1$ is preferably hydrogen or methyl, most preferably, however, hydrogen.

m is 1, 2 or 3, preferably 1 or 2 and most preferably 1 and n is 1 or 2, preferably 2.

The compounds of formula (1) are advantageously prepared by eliminating HX in the intermediate of the formula

$$R_f\!\left(CH_2\underset{\underset{\displaystyle R_1}{|}}{CH}\right)_m S(O)_n-\underset{\underset{\displaystyle H}{|}}{CH}-\underset{\underset{\displaystyle X}{|}}{CH_2} \qquad (2)$$

wherein X is a leaving group, and $R_f$, $R_1$, m and n are as defined above.

Suitable leaving groups include halogen such as chlorine, bromine or iodine, hydroxyl, lower alkoxy, lower acyloxy, alkali metal sulfato and the like. Preferably, X is hydroxyl, chlorine, alkali metal sulfato or acetoxy, most preferably hydroxyl, chlorine or acetoxy.

The term, "lower", as used herein, refers to groups having 1 to 5 carbon atoms in the alkyl moiety thereof.

Advantageously, the elimination reaction is conducted at temperatures between about 0 and 100°C, in the presence of an inert solvent or solvent mixture, such as an acetone/water mixture, tetrahydrofuran, din-propyl ether, and the like, and where XH is an acid, preferably in the presence of an acid acceptor, such as triethylamine, N,N-dimethyl benzylamine, sodium hydroxide and the like.

Those compounds of formula (1) wherein n is 1 or 2 can be prepared from the corresponding thioether of the formula

$$R_f\!\left(CH_2\underset{\underset{\displaystyle R_1}{|}}{CH}\right)_m S-CH_2CH_2X \qquad (3)$$

wherein $R_f$, $R_1$, m and X are as defined above, by oxidation with an organic or inorganic peroxide, preferably hydrogen peroxide, in a solvent, such as acetic acid, at a temperature between about 10 and 100°C. If the sulfoxide is desired, then advantageously about one mole of peroxide is reacted per mole of the compound of formula (3) and if the sulfoxide product is desired, then at least 2 moles of peroxide is advantageously added for each mole of the compound of formula (3) present.

An excess of peroxide in the latter case is often desirable to insure an optimized yield of the sulfoxide product.

The compounds of formula (3) can advantageously be prepared by a variety of techniques.

For example, compounds of formula (3), wherein X is lower acyloxy or halogen, can advantageously be prepared by reacting a mercaptan of the formula

$$R_f\text{-}(CH_2\underset{\underset{R_1}{|}}{CH})_m\text{—}SH \qquad (4)$$

wherein $R_f$, $R_1$ and m are as defined above with a vinyl derivative of the formula

$$CH_2{=}CH\text{—}X \qquad (5)$$

wherein X is lower acyloxy or halogen, in the presence of a free radical initiator, preferably an azo compound such as azo-bis-2-methylpropionitrile, optionally in the presence of an inert diluent or solvent, at a temperature between about −100°C and 100°C.

As an alternate method, the aforementioned mercaptan of formula (4) can be reacted with a compound of the formula

$$X_1\text{—}CH_2CH_2\text{—}X \qquad (6)$$

wherein $X_1$ is chloro or bromo, and X is halogen, hydroxyl, lower acyloxy or lower alkoxy, in the presence of a basic agent, such as an alkali metal hydroxide, advantageously in the presence of inert diluent, at a temperature between about 0°C to 100°C. Optionally, the reaction can take place in a heterogeneous aqueous/organic phase in the presence of a phase transfer catalyst, such as benzyl trimethyl ammonium chloride, tricaprylmethyl ammonium chloride, didodecyl dimethyl ammonium bromide or the like.

The perfluoroalkyl alkylene vinyl sulfoxides and sulfones according to formula (1) can be telomerized or polymerized alone or in combination with conventional monomers to obtain polymeric product having advantageous oil and water repellent properties. Said telomers or polymers thus contain structure units of the formula

$$\text{—}(CH_2\text{—}\underset{\underset{\underset{\underset{\underset{R_1}{|}}{(CH\text{—}CH_2)_m\text{—}R_f}}{|}}{S(O)n}}{|}}{CH})\text{—} \qquad (7)$$

wherein $R_f$, $R_1$, m and n are as defined.

Thus, for example, the compounds of formula (1) can be caused to form polymers or copolymers, by polymerization techniques known per se. Thus, the compounds of formula (1) can, for example, be telomerized or polymerized in solution, e.g. in the presence of an organic solvent, such as benzene, toluene, xylene or the like, in the presence of actinic light, e.g. ultraviolet light, or a free radical forming catalyst, such as azo-bis-2-methylpropionitrile.

If desired, the compounds of formula (1) may be copolymerized with other monomers, including vinyl compounds, such as vinyl acetate and vinyl chloride; alkenes, such as ethylene, isopropylene, styrene; acrylates and methacrylates such as methyl acrylate, butyl acrylate, methyl methacrylate and the like, etc. The nature of such other monomers is not critical, and are sometimes found to be advantageous extenders of the desirable oleophobic and hydrophobic properties exhibited by the polymers of the monomers of formula (1) when coated onto appropriate substrates.

Suitable substrates include cellulosic and natural and synthetic polyamide materials include, for example, paper articles, paper pulp, cotton, regenerated cellulose, jute and the like, leather, feathers, nylon fibers, etc.

Thus, materials treated may be in the form of fibers, pulp, film, solution or yarn fabrics. The materials are advantageously coated with the polymer or copolymer of the compound of formula (1) to impart oil and water repellency to said materials.

Alternatively, a solution or emulsion of the compound of formula (1) may be applied to the surface of the material by any convenient means, such as dipping, padding or the like, and polymerized in situ, optionally in the presence of an alkaline catalyst, such as an alkali metal hydroxide, and a free radical catalyst, if desired, to form a polymer firmly bonded to the material substrate.

Suitable solvents for the compounds of formula (1) and the telomers, polymers and copolymers thereof include conventional organic solvents such as ketones, e.g. acetone, hydrocarbons, e.g. benzene, toluene and xylene, ethers e.g. tetrahydrofuran, haloalkanes, e.g. chlorinated ethanes, fluorinated ethanes and the like. Also aqueous and aqueous/organic emulsions may be employed by the use of conventional surfactants and the like. Application temperatures may vary depending upon the application technique, but will ordinarily range between about 10°C and 120°C, under ambient or superatmospheric pressure.

The following examples are for illustrative purposes only and are not intended to limit the scope of the invention.

Example 1

$$F_{13}C_6CH_2CH_2SO_2CH_2CH_2O_2CCH_3 + OH^{\ominus} \xrightarrow[pH\ 10—11]{CH_3COCH_3,\ 23°C} F_{13}C_6CH_2CH_2SO_2CH{=}CH_2 + CH_3CO_2^{\ominus} + H_2O$$

2-(Perfluorohexyl)ethyl Vinyl Sulfone

2-(Perfluorohexyl)ethyl-2-acetoxyethyl sulfone (10.27 g, 0206 mole), 100 ml of acetone and 10 ml of water was stirred by magnet bar in an Erlenmeyer flask, fitted with an hydrogen ion electrode attached to a Sargent-Welch pH-meter. At 23°C the pH-value was brought to 10.4 by adding 0.25 M NaOH solution (62 ml) and kept in the pH-range of 10.57—11.07 (44 ml of base) during 8 hours at 22—23°C. The solution remained slightly cloudy throughout. On standing overnight the pH-value dropped to 8.97; thus, base (2.00 ml) was added at a pH-value of 10.2—11.4, until it was constant at 10.9. Acetone was evaporated off in an open beaker at 55—62°C, the mixture cooled to 25°C and solid product precipitated, 8.60 g (95%) of 2-(perfluorohexyl)ethyl vinyl sulfone (melting point 58—60°C). The product was recrystallized from hot carbon tetrachloride (8.0 g in 40 ml, filtered hot) and the melting point of the pure product raised to 60—61°C. An infrared spectrum (Mineral oil mull) showed bands at 3060, 3030 (HC=), 1610 (CH=CH), 1090, 1075 ($SO_2$), 990, (960) and 920 $cm^{-1}$. 1H—NMR-spectrum was consistent with the structure of the product $F_{13}C_6CH_{2A}CH_{2B}SO_2CH_C{=}CH_DH_E$: at 2.60 ppm, a complex multiplet, 2 protons ($CH_{2B}$); at 3.24 ppm, complex multiplet, 2 protons ($CH_{2B}$); at 6.24 ppm, 2 sets of resonances, 3 protons, ($CH_C{=}CH_DH_E$) δ-values).

Anal.:

| | | | | |
|---|---|---|---|---|
| Calcd. for $C_{10}H_7F_{13}O_2S$: | C, 27.4; | H, 1.61; | F, 56.4; | S, 7.32. |
| Found: | C, 27.4; | H, 1.6; | F, 56.2; | S, 7.7. |

Example 2

$$F_{17}C_8CH_2CH_2SO_2CH_2CH_2Cl + (H_5C_2)_3N \xrightarrow{THF,\ 45°C} F_{17}C_8CH_2CH_2SO_2CH{=}CH_2 + (H_5C_3)_2N{\cdot}HCl$$

2-(Perfluorooctyl)ethyl Vinyl Sulfone

2-(Perfluorooctyl)ethyl-2-chloroethyl sulfone (5.75 g, 10.0 mmole) was dissolved in 40 ml of tetrahydrofuran (THF) at 45°C, while stirring in a constant temperature oil bath Triethylamine (1.52 g, 2.10 ml, 15.0 mmole) in 10 ml of THF was added and an exotherm carried the temperature to 51°C. A precipitate of the white solid triethylammonium chloride immediately began to form. After 1 hour at 45°C the slurry was filtered while hot (Buchner funnel), rinsed with diethyl ether twice, and the clear filtrate evaporated on a rotary evaporator. The dry solid $(H_5C_2)_3N.HCl$ weighed 1.35 g, 98.8% of theory. The evaporated solid 2-(perfluoro-octyl)ethyl vinyl sulfone was taken to 40°C/15 mm and weighed 5.38 g (100% melting point 93—94.5°C). The sulfone was heated further to 155°C without evidence of decomposition. The sulfone was soluble in chloroform and insoluble in carbon tetrachloride, benzene or ligroine (bp 60—70°C). The sulfone dissolved in hot benzene, but was insoluble in hot ligroine. A 1.00 g sample was dissolved in 8.0 ml of benzene by heating on a steam bath, with care to prevent foaming over; 8.0 ml of ligroine was added and the clear, colorless solution when cool gave 0.77 g of solid (melting point 95—96°C). When concentrated to 4.0 ml volume a fraction of 0.11 g (melting point 96—97°C) was obtained. The original sample gave a single spot in TLC analysis. The infrared spectrum was identical to that of the $C_6$-homolog of Example 1. The 1H—NMR-spectrum was consistent with the proposed structure and resembled closely that obtained from said $C_6$-homolog.

Anal.:

| | | | | |
|---|---|---|---|---|
| Calcd. for $C_{12}H_7F_{17}O_2S$: | C, 26.8; | H, 7.06; | F, 60.0; | S, 5.96. |
| Found: | C, 26.5; | H, 1.5; | F, 60.2; | S, 6.8. |

Example 3

2-(Perfluorohexyl)ethyl Vinyl Sulfone

2-(Perfluorohexyl)ethyl 2-chloroethyl sulfone (4.75 g, 10.0 mmole) was dissolved in 25 ml of THF at 30°C and triethylamine (1.52 g, 15.0 mmole) in 10 ml of THF was added in one minute. A white precipitate formed, and was filtered (1.37 g). The solvent was stripped carefully, as some foaming occurred.

The sulfone (4.33 g 100% of theory), had a melting point of 60—62°C. A 1.00 g-sample was recrystallized from 15 ml of hot ligroine (bp 60—70°C). Fractions obtained were 0.90 g (melting point 60—61°C) and 0.07 g (melting point 60—61°C). TLC analysis of the original sample gave a single spot.

## Example 4

2-(Perfluorodecyl)ethyl Vinyl Sulfone

2-(Perfluorodecyl)ethyl 2-chloroethyl sulfone (3.37 g, 5.00 mmole) and tetrahydrofuran (25 ml) were stirred at 50°C and triethylamine (1.01 g, 10.0 mmole) was added, giving an exotherm to 52°C. After a half hour at 50°C, the reaction mixture was evaporated off and the white solid (3.83 g) was suspended and stirred in water (50 ml). The solid was collected and dried (2.96 g, melting point 117—121°C). A sample recrystallized from benzene and acetone gave 2.18 g of a white solid (melting point 123—125°C). An IR spectrum (Nujol mull) gave bands at 3120, 3040, 1600, 970 and 930 $cm^{-1}$, characteristic of 2-(perfluorodecyl)-ethyl vinyl sulfone. The yield was 92.7% of theory.

## Example 5

$$F_{21}C_{10}CH_2CH_2SCH_2CH_2Cl + 2H_2O_2 \xrightarrow{CH_3CO_2H} F_{21}C_{10}CH_2CH_2SO_2CH_2CH_2Cl$$

Preparation of 2-(Perfluorohexyl)ethyl 2-chloroethyl Sulfone

5-(Perfluorohexyl)-3-thia-1-chloropentane (6.16 g, 13.9 mmole) was stirred by magnet bar and dissolved in 5 ml of acetic acid at 50°C. Hydrogen peroxide (4.42 g, 39.0 mmole, Fisher Certified, 30% aqueous solution) in 10 ml of acetic acid was added dropwise. The reaction mixture was heated to 70°C for 2 hours. A sample had a melting point of 104—105.5°C. After 80 minutes at 70°C and 30 minutes at 90°C, the mixture was evaporated off at room temperature, to yield 6.48 g of a white solid, (98% of theory, melting point 101—103°C). Recrystallized from benzene (40 ml) at 68°C gave 6.09 g product of melting point of 105—105.5°C, 0.22 g and 0.04 g of products with melting point of 105—106°C. An IR spectrum ($HCCl_3$) gave bands at 1430, 1400, 1065, 1050, 1015, 955, 935 and 900 $cm^{-1}$ that were characteristic of the substance. An 1H—NMR spectrum gave proton resonances at 2.80 ppm, a complex pattern, 2 protons, $CH_{2A}$; at 3.42 ppm, a complex pattern, 4 protons, $CH_{2B}$ and $CH_{2C}$; and at 3.97 ppmm a triplet, 2 protons $CH_{2D}$; (of $F_{21}C_{10}CH_{2A}CH_{2B}SO_2CH_{2C}CH_{2D}Cl$, δ-values).

Anal.:

| | | | | | |
|---|---|---|---|---|---|
| Calcd. for $C_{10}H_8F_{13}ClO_2S$: | C, 25.3; | H, 1.7; | F, 52.0; | Cl, 7.47; | S, 7.0. |
| Found: | C, 25.2; | H, 1.4; | F, 52.1; | Cl, 7.2; | S, 7.0. |

## Example 6

Preparation of 2-(Perfluorooctyl)ethyl 2-Chloroethyl Sulfone

5-(Perfluorooctyl)-3-thia-1-chloropentane (5.18 g, 9.54 mmole) in 10 ml of acetic acid and hydrogen peroxide (3.40 g, 30.0 mmole) in 5 ml acetic acid were allowed to react under conditions used in Example 5. The dry product weighed 5.34 g (97.4% of theory, melting point 130—131°C). A sample recrystallized from benzene had a melting point of 132—132.5°C. An IR spectrum ($CHCl_3$ slurry) gave bands at 1435, 1400, 1090, 1070, 1055, 1020 and 940 $cm^{-1}$.

Anal.:

| | | | | | |
|---|---|---|---|---|---|
| Calcd. for $C_{12}H_8F_{17}ClO_2S$: | C, 25.08; | H, 1.40; | F, 56.20; | Cl, 6.17; | S, 5.58. |
| Found: | C, 25.0; | H, 1.4; | F, 56.2; | Cl, 6.3; | S, 6.0. |

## Example 7

2-(Perfluorodecyl)ethyl 2-Chloroethyl Sulfone

5-(Perfluorodecyl)-3-thia-1-chloropentane (5.00 g, 7.78 mmole) in 10 ml of acetic acid and hydrogen peroxide (3.40 g, 30.0 mmole) in 5 ml of acetic acid were allowed to react under conditions used in Example 5, but for reaction at 90°C for 15 hours. 5 g (95%) of a white solid formed in the reaction that was scraped out of the flask (melting point 147—148°C). A sample recrystallized from benzene had a melting point of 147—149°C. Sintering was observed in both samples at about 141—143°C.

## Example 8

$$F_{13}C_6CH_2CH_2SH + ClCH_2CH_2Cl + NaOH \longrightarrow$$
$$F_{13}C_6CH_2CH_2SCH_2CH_2Cl + (F_{13}C_6CH_2CH_2SCH_2CH_2SCH_2CH_2C_6F_{13}) + NaCl$$

5-(Perfluorohexyl)-3-thia-1-chloropentane

A solution of 2-(perfluorohexyl)ethylthiol (7.60 g, 20.0 mmole) in 50 ml of water containing sodium hydroxide (0.80 g, 20 mmole) was added during 1.5 hours to 1,2-dichloroethane (61.75 g, 62.4 mmole, 50 ml) and "Aliquat 336" to (0.13 g, 0.29 mmole, General Mills tricaprylmethylammonium chloride), while stirring by means of a paddle stirrer in a 200 ml, round bottom flask at 25—27°C. Two liquid phases were present. After 5 hours a sample of the organic layer was dried ($MgSO_4$) and CG analysis [6ft SE 30 silicone oil on Chromosorb WA, temperature programmed from 150—180°C, 33.5 ml/min of helium flow] showed 1,2-dichloroethane (93.5%), title compound (6.32%) and a residue of unknown structure (0.3%). The ratio of

the last two peaks was 97.6/2.4. After 6 hours, the layers were separated, the organic layer dried and distilled in a 16-inch spinning band column, to give 58 g of 1,2-dichloroethane and 7.15 g (80.7% of theory) of 5-(perfluorohexyl)-3-thia-1-chloropentane, boiling point 80—81°C/1.75 mm, $n_D^{25}$ 1.3717. A crystalline residue (0.81 g) remained.

Anal.:

| | | | | | |
|---|---|---|---|---|---|
| Calcd. for $C_{10}H_8F_{13}ClS$: | C, 27.1; | H, 1.8; | F, 55.8; | Cl, 8.0; | S, 7.2. |
| Found: | C, 27.4; | H, 1.7; | F, 55.7; | Cl, 8.0; | S, 7.5. |

### Example 9

5-(Perfluorooctyl)-3-thia-1-chloropentane

2-(Perfluorooctyl)ethylthiol (48.75 g, 0.1018 mole; 0.78% $C_6$, 95.6% $C_8$ and 0.84% $C_{10}$ $R_F$ groups) was added, while stirring under nitrogen, to sodium hydroxide (4.58 g, 0.165 mole) in water (75 ml). The thick slurry was diluted with water (100 ml) and added in portions during a half hour to 1,2-dichloro-ethane (3.09 g, 3.12 mole, 250 ml) and "Aliquat 336" (0.50 g, 1.1 mmole) while stirring in a 500-ml round bottom 3-necked flask fitted with a paddle stirrer, nitrogen purge inlet tube and condenser. An exotherm carried the temperature from 24 to 28°C. After 7 hours at ambient temperature the layers were separated, and the aqueous layer extracted with 1,2-dichloroethane (20 ml). The organic layers were combined and filtered from an insoluble solid (3.19 g, melting point 68—72°C). Unreacted 1,2-dichloroethane was removed by distillation and the residual oil (81.45 g) was distilled in a 16-inch spinning band column. Fractions obtained were: I, boiling point 98—104°C/3.8 mm, 1.73 g; II, boiling point 104—105°C/3.8 mm, 43.25 g, melting point 39—40°C; and III, boiling point 85°C/0.75—0.30 mm, 2.10 g. The total yield of $C_8F_{17}CH_2CH_2SCH_2CH_2Cl$ was 86.8% of theory. A residue of 3.02 g remained in the pot flask. In TLC analysis Fractions I, II and III gave only $C_8F_{17}CH_2CH_2SCH_2CH_2Cl$.

Anal.:

| | | | | | |
|---|---|---|---|---|---|
| Calcd. for $C_{12}H_8F_{17}ClS$: | C, 26.6; | H, 1.48; | F, 59.5; | Cl, 6.9; | S, 5.9. |
| Found: | C, 26.6; | H, 1.6; | F, 59.4; | Cl, 6.9; | S, 6.5. |

### Example 10

5-(Perfluorodecyl)-3-thia-1-chloropentane

($R_f$=$C_4$, 0.8%; $C_6$, 34.1%; $C_8$, 35.8%; $C_{10}$, 22.7%; $C_{12}$, 6.5% and $C_{14}$, 0.5%). A 2-perfluorodecylethylthiol mixture (46.70 g, 0.10 mole, as listed above), a solution of sodium hydroxide (4.60 g, 0.115 mole) in 200 ml of water and "Aliquat 336" (0.23 g, 0.52 mmole) were stirred under nitrogen purge as in the reactions described above, while 1,2-dichloroethane (450.8 g, 4.55 mole) was added at 25°C. The two layers were stirred for 6.5 hours, the last two hours heated to 50°C. Work-up of the reaction product mixture gave distilled fractions as follows: I, boiling point 70—82°C/2.5 mm, 14.64 g; II, boiling point 80—96°C/1.5 mm, 5.37 g; III, boiling point 91—102°C/2.5 mm, 12.93 g; VI, boiling point 92—106°C/0.35—0.30 mm, 6.65; and residue, 2.50 g. Fractions I to III were mixtures of $C_4$ to $C_8$ (chiefly), while fraction IV was chiefly $C_{10}F_{21}CH_2CH_2SCH_2CH_2CH_2Cl$. An insoluble solid 6.86 g melting point 105—115°C) was filtered from the organic layer before distillation.

### Example 11

5-(Perfluorooctyl)-3-thia-1-chloropentane

2-(Perfluorooctyl)ethylthiol 4.80 g, 10.0 mmole), 1,2-dichloroethane (29.67 g, 300 mmole) and 10 ml of water were stirred by magnet bar under nitrogen at ambient temperature, while sodium hydroxide (0.67 g, 16.7 mmole) in 20 ml of water was added dropwise during a half hour. An exotherm carried the temperature from 24.0°C to 25.2°C. A thick, white slurry was formed. After 5 hours of stirring at 23.5°, the white slurry had not separated into two liquid layers as in the procedures used above. Stirring was continued for 29 hours and a sample removed for analysis. After 39 hours the thick white paste was made acidic by the addition of 5.0 ml of 6N hydrochlorid acid, and the white solid collected on a Buchner funnel, was washed with water. When dry, the solid weighed 3.45 g (melting point 83—88°C), 70% of theory for $C_8F_{17}CH_2CH_2SCH_2CH_2SCH_2CH_2C_8F_{17}$). A 1.00 g sample was recrystallized from hot chloroform, that was first filtered while hot, from insoluble salt and yielded 0.54 g, product of a melting point of 92—94°C. The filtrate deposited 0.26 g of less pure solid. The original filtrate containing water and 1,2-dichloroethane (above) was separated into layers, and the organic layer evaporated off to give 1.18 g of a white solid of a melting point of 30—33°C (21.7% of theory for $C_8F_{17}CH_2CH_2SCH_2CH_2Cl$).

### Example 12

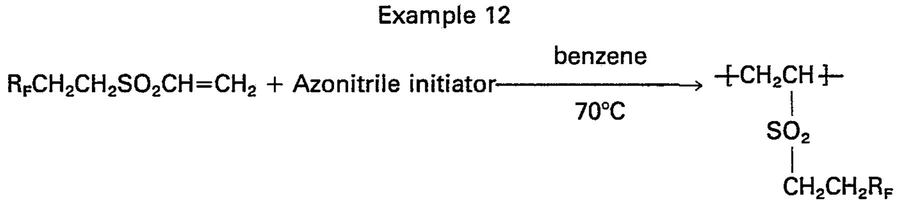

Polymerization of 2-(Perfluoroalkyl)ethyl Vinyl Sulfones

2-(Perfluoroalkyl)ethyl vinyl sulfone (1.00 g, 1.86 mmole, $R_F$=0.78%, $C_6F_{13}$, 95.6% $C_8F_{17}$ and 0.84% $C_{10}F_{21}$) and 0.100 g (0.61 mmole) of azo-bis-2-methylpropionitrile were heated under nitrogen in 20 ml of benzene while stirring by magnet bar in a flask under a reflux condenser. The initially clear solution became cloudy and formed a white suspension during the first 5 hours. After 17 hours of reaction the slurry was filtered, rinsed twice with benzene and dried to yield 0.75 g of the polymer (melting point (96—115°C. An IR spectrum showed no vinyl absorption bands. Evaporation of the filtrate gave 0.32 g of solid solid.

Example 13

Application of poly-2-(Perfluoroalkyl)ethyl Vinyl Sulfone to Paper

A solution of 0.1004 g of polymer of Example 12 in 5.0 ml of acetone was prepared which appeared as slightly cloudy. It was filtered through a Whatman filter paper and dried. The filtrate remained cloudy. The filtrate was similarly drawn through another filter paper, and dried, with an increase in weight of 9.8%, both papers were highly repellent to water, to cooking oil and to toluene. The drops formed very high contact angle on the surface, did not spread or wet the surface and did not penetrate the paper. This results indicate that the polymer gave a coating on paper that was both hydrophobic and oleophobic.

Example 14

$$F_{17}C_8CH_2CH_2SH + C_2H_3O_2CCH_3 \xrightarrow[70°C]{\text{Azonitrile initiator}} F_{17}C_8CH_2CH_2SCH_2CH_2O_2CCH_3$$

5-(Perfluorooctyl)ethyl-3-thiapentyl-1-acetate

A glass pressure tube was charged with 2-(perfluorooctyl)-ethylthiol (24.0 g, 50.0 mmole), vinyl acetate (redistilled, 4.30 g, 55.0 mmole) and azo-bis-2-methylpropionitrile (0.0821 g, 0.500 mmole). The tube was cooled to −78°C, evacuated and filled with nitrogen three times, and sealed. The tube was heated in a stirred oil bath at 70°C for 12 hours, cooled and the cloudy, colorless liquid distilled. Forerun, boiling point 93—110°C/1.75 mm, 2.51 g, that contained unreacted thiol (0.71 g) and the desired acetate product (0.71 g), was collected. Then, the acetate, boiling point 106—108°C/0.75 mm, $n^{25}_D$ 1/3628, 95.4% pure by GC, was taken. The total yield of the acetate was 25.4 g, 89.7% of theory. The residue (0.66 g) was chiefly initiator-derived products. The cold trap contained 0.96 g of vinyl acetate and a little diethyl ether used to rinse the reaction vessel. The infrared spectrum of the acetate showed the >C=O band at 1740 $cm^{-1}$. The 1H—NMR spectrum gave proton resonances at 2.00 ppm, a singlet, 3 protons, $CH_3$; at 2.75 ppm, a complex multiplet, 6 protons, $(CH_2)_2SCH_2$; and at 4.20 ppm, triplet, (J=7 Hz), 2 protons, $CH_2O_2C$—. These data (δ-values) are in accord with the structure of the little compound.

Anal.:

| | | | |
|---|---|---|---|
| Calcd. for $C_{14}H_{11}F_{17}O_2S$: | C, 29.7; | H, 2.0; | F, 57.0. |
| Found: | C, 29.8; | H, 2.0; | F, 57.7. |

Example 15

5-(Perfluorohexyl)ethyl-3-thiapentyl-1-acetate

A 100 ml, round bottom flask was charged with 2-(perfluorohexyl)ethylthiol (redistilled, boiling point 73°C/32 mm, 26.61 g, 70.0 mmole) and azo-bis-2-methylpropionitrile (0.150 g, 0.91 mmole), purged with nitrogen, and heated in a bath at 65—75°C while stirring by magnet bar. Vinyl acetate (7.14 g, 82.5 mmole) was added during 40 minutes at 65—72°C, and stirring under nitrogen continued at 75°C for 17 hours. The reaction mixture was distilled, giving a forerun boiling point 68—100°C/11 mm, 1.00 g (mostly unreacted thiol; and the desired acetate boiling point 108—113°C/3.6 mm, 30.2 g, $n^{25}_D$ 1.3692. GC analysis showed a purity of 99.7%. A residue of 0.77 g and cold trap liquid of 0.51 g were obtained. The total yield of acetate was 30.7 g, or 99.7% of theory. Infrared and NMR spectra were consistent.

Anal.:

| | | | | |
|---|---|---|---|---|
| Calcd. for $C_{12}H_{11}F_{13}O_2S$: | C, 30.9; | H, 2.38; | F, 53.0; | S, 6.88. |
| Found: | C, 31.1; | H, 2.3; | F, 53.4; | S, 6.7. |

Example 16

$$F_{13}C_6CH_2CH_2SCH_2CH_2O_2CCH_3 + H_2O_2 \xrightarrow[35—50°C]{CH_3CO_2H/(CH_3CO)_2O} F_{13}C_6CH_2CH_2SO_2CH_2CH_2O_2CCH_3$$

2-(Perfluorohexyl)ethyl 2-Acetoxyethyl Sulfone

To 5-(Perfluorohexyl)ethyl-3-thiapentan-1-acetate (46.85 g, 0.100 mole) stirred by magent bar, was added acetic acid (75 ml) and acetic anhydride (25 ml) at 35°C. While cooling with a bath at 20°C hydrogen

peroxide (8.16 g, 0.24 mole, 30.1 ml of 27% by weight, aqueous solution) was added in part (10 ml) at 35—42°C during a half hour. Exothermic reaction occurred, and the bath temperature was raised to 38—40°C. Hydrogen peroxide (remaining 20 ml) was added slowly during one hour, while raising the bath temperature to 60°C. The mixture was stirred at 60°C for 15 hours. A sample that was precipitated into water gave a thick gel. Severe foaming occurred when evaporation in a rotary evaporator was attempted at reduced pressuure. Thus, the solvent was distilled with great care in a packed column, boiling point 30°C/25 mm. The residue 51.03 g, was dried in vacuo over phosphoric anhydride at 20 mm for 18 hours, and weighed 43.58 g, 88.2% of theory for 2-(perfluorohexyl)ethyl 2-acetoxyethyl sulfone. A sample melted at 62—64°C, and recrystallized from carbon tetrachloride at 63—64°C'. The infrared spectrum gave bands at 3450—3200 (weak, OH), 1750 (C=O), and at 1300—1250, 1095, 1080, 1050 and 960 $cm^{-1}$. The 1H—NMR spectrum of $F_{17}C_6CH_{2B}CH_{2C}SO_2CH_{2D}CH_{2E}O_2CCH_{3A}$ gave proton resonances at 2.10 ppm, ($CH_{3A}$); at 2.70 ppm, broad, complex multiplet, 2 protons ($CH_{2B}$); at 3.34 ppm, broad, complex multiplet, 4 protons ($CH_{2C}$ and $CH_{2D}$); at 4.15 ppm, a triplet, about 0.2 proton ($CH_{2E}OH$); and at 4.52 ppm, a triplet, about 0.8 proton $CH_{2E}OAc$ (δ-values). Some hydrolysis of the acetate ester was probably responsible for the formation of alcohol in the sample.

Anal.:

| | | | | |
|---|---|---|---|---|
| Calcd. for $C_{12}H_{11}F_{13}O_4S$: | C, 28.9; | H, 2.2; | F, 49.6; | S, 6.4. |
| Found: | C, 28.4; | H, 2.1; | F, 50.1; | S, 7.1. |

## Example 17

$$R_FCH_2CH_2SH + ClCH_2CH_2OH \xrightarrow{NaOH} R_FCH_2CH_2SCH_2CH_2OH + NaCl$$

In a 300 ml, round bottom 3-neck flask fitted with a nitrogen inlet, a constant rate dropping funnel and immersed in a constant temperature oil bath at 60°C, was placed $F_{13}C_6CH_2CH_2SH$ ($C_6F_{13}$, 97.6%; $C_8F_{17}$, 1.3%; 38.0 g, 23.0 ml, 0.100 mole), 2-chloroethanol (16.1 g, 0.200 mole), tridecylmethylammonium chloride (0.25 g, 0.56 mole, "Aliquat 336", General Mills), and benzene (25 ml). A solution of sodium hydroxide (6.00 g, 0.150 mole) in water (50 ml) was added slowly during a half hour, while stirring by means of a magnet bar. At the beginning a purple color appeared which disappeared after 2 hours. The aqueous layer had a pH-value of 8—9. A sample was taken after 6 hours, and stirring continued for 24 hours total time. A second sample was taken. The samples were converted to trimethylsilyl derivatives by reaction with BSTFA (n-o-bis(trimethylsilyl)trifluoro acetamide, standard silylating agent) and GC analysis run: 5-ft "W—98" silicone oil, 43 ml/min of helium, 150°C for five minutes, then 250°C. Only peaks for homologs of the product were present at 9.6, 10.6 and 11.0 minutes. The layers were separated, the aqueous layer extracted twice with benzene (10 ml), rinsed with water and dried over $MgSO_4$.

The benzene was removed by rotary evaporator, leaving 40.1 g of viscous oil (94.5% of theory not including samples) for $C_6F_{13}CH_2CH_2SCH_2CH_2OH$. An 1H—NMR spectrum was consistent with the formula $C_6F_{13}CH_{2A}CH_{2B}SCH_{2C}CH_{2D}OH$, showing proton resonances at 2.38 ppm, a complex multiplet, 2 protons area, $CH_{2A}$; at 2.75 ppm, a complex multiplet, 4 protons, $CH_{2B}$ $CH_{2(C)}$; and at 3.67 ppm, a pseudo-q, 2 protons, $CH_{2(D)}$ (δ-values).

Anal.:

| | | | | |
|---|---|---|---|---|
| Calcd. for $C_{10}H_9F_{13}OS$: | C, 28.3; | H, 2.14; | F, 58.2; | S, 7.56. |
| Found: | C, 28.6; | H, 2.14; | F, 58.3; | S, 7.7 |

## Example 18

5-(Perfluoroalkyl)-3-thia-pentanol

In the same manner as in Example 17, $R_fCH_2CH_2SH$ ($R_F$=$C_6F_{13}$, 12.7%; $C_8F_{17}$, 82.7%; $C_{10}F_{21}$, 3.3%; and $C_{12}H_{23}$, 0.96%; 65.0 g, 0.140 mole), 2-chloroethanol (22.54 g, 0.2799 mole), "Aliquat 336" (0.30 g, 0.67 mmole), sodium hydroxide (8.44 g, 0.211 mole), benzene (50 ml) and water (100 ml) at 60°C, gave $R_fCH_2CH_2SCH_2CH_2OH$ ($R_F$=$C_6$, $C_8$, $C_{10}$ and $C_{12}$) as a precipitated white solid. When dried ($P_2O_5$) in vacuo, it weighed 69.11 g and contained sodium chloride that would not dissolve by water washing. A portion (10 g) was extracted into hot carbon tetrachloride, filtered from insoluble salt (0.45 g), and cooled; 6.65 g, melting point 68—71°C, and subsequent fractions, a total of 9.22 g, were recovered. The remaining impure product was dissolved in anhydrous diethyl ether, filtered (3.67 g insoluble solid), and the ether removed; 53.8 g of solid was obtained that was used for analysis and subsequent reactions. GC analysis gave title compounds: $C_6$, 10.0%; $C_8$, 84.3%, $C_{10}$, 4.8% and $C_{12}$, 0.96%. An 1H—NMR spectrum was consistent with the structure of $R_fCH_2CH_2SCH_2CH_2OH$. The total product recovered was 62.6 g 85.3% of theory.

## Example 19

5-(Perfluorohexyl)-3-thia-pentanol Acetate

$C_6F_{13}CH_2CH_2SCH_2CH_2OH$ (40.1 g, 0.094 mole, undistilled), acetic anhydride (16.2 g, 0.15 mole) and pyridine (0.98 g, 12.4 mmole) was heated at 65—66°C for 4 hours and 89°C for 17 hours under total reflux. A

sample after 4 hours showed incomplete reaction and after 17 hours, only a trace of $C_6F_{13}CH_2CH_2SCH_2CH_2OH$ was present (CG analysis). The product was washed with saturated sodium bicarbonate (20 ml), extracted into ether, dried and the solvent removed by distillation; wt. 47.77 g. Total product was 49.3 g, 100% of theory. 1H—NMR was consistent with the structure $C_6F_{13}CH_{2A}CH_{2B}SCH_{2C}CH_{2D}O_2CCH_3$ proton resonances at 2.03 ppm, 3 protons, s, $CH_3$; at 2.42 ppm, multiplet, 2 protons, $CH_{2A}$; at 2.78 ppm, multiplet, 4 protons, $CH_{2A}$; at 2.78 ppm, multiplet, 4 protons, $CH_{2B}$ and $CH_{2C}$; and at 4.24 ppm, t, 2 protons $CH_{2D}$ (δ-values).

Anal.:

| | | | | |
|---|---|---|---|---|
| Calcd. for $C_{12}H_{11}F_{13}SO_2$: | C, 30.9; | H, 2.38; | F, 53.0; | S, 6.88. |
| Found: | C, 31.1; | H, 2.3; | F, 53.4; | S, 6.7. |

### Example 20

5-(Perfluorooctyl)-3-thia-pentanol Acetate

$C_8F_{17}CH_2CH_2SCH_2CH_2OH$ (60 g, 0.11 mole, crude product, $C_8$=95.6%) and acetic anhydride (15 g, 0.15 mole) was heated at 80°C for 17 hours, the volatile material removed by rotary evaporator; weight of product 64.4 g, 100% of theory. The IR spectrum shows no band for OH but a strong carbonyl band at 1735 $cm^{-1}$. GC showed traces of impurities and chiefly the desired acetate. 1H—NMR was consistent with the structure.

Anal.:

| | | | | |
|---|---|---|---|---|
| Calcd. for $C_{14}H_{11}F_{17}SO_2$: | C, 29.7; | H, 1.96; | F, 57.0; | S, 5.66. |
| Found: | C, 29.7; | H, 2.0; | F, 55.8. | |

### Example 21

2-(Perfluorooctyl)ethyl-2-acetoxyethyl Sulfone

The product of Example 20 (6.00 g, 0.0106 mole, crude product) was dissolved in acetone (50 ml), and water (10 ml) was added, followed by 3 ml of 0.3M ammonium molybdate solution, at 23°C.

A precipitate formed that dissolved when hydrogen peroxide (1.70 g, 0.050 mole, 6.3 ml, 27%) was added in portions during 15 minutes. The temperature rose to 31°C and the mixture, while stirring by magnet bar, set up to a crystalline solid mass. After 1 hour at 30°C, the mixture was heated to 46—48°C for 1 hour, and cooled to 25°C. A sample gave a melting point of 96—100°C (decomposed). The reaction mixture was worked up by adding 50 ml of water and extracting into chloroform (50 ml). The clear, colorless chloroform solution was dried ($MgSO_4$), filtered and evaporated off in rotary evaporator to 50°C/15 mm. The solvent came off at first, but then the product began to sublime into the condenser. The material was dissolved out with acetone and evaporated off, 4.55 g, 72% of theory, melting point 93—96°C (capillary tube placed in the bath at 93°C).

IR spectrum showed a carbonyl band at 1740 $cm^{-1}$, and bands at 1090, 1045, 960; 845, 800; 775, 750, 710; 650, 610, 590, 580, 560, 530, 500 $cm^{-1}$.

Anal.:

| | | | | |
|---|---|---|---|---|
| Calcd. for $C_{14}H_{11}F_{17}SO_4$: | C, 28.1; | H, 1.85; | F, 54.0; | S, 5.36. |
| Found: | C, 28.2; | H, 1.7; | F, 54.2; | S, 5.8. |

## Claims

1. A compound of the formula

$$R_f\!\left(CH_2-\underset{\displaystyle R_1}{\underset{|}{C}H}\right)_m S(O)_n-CH=CH_2 \qquad (1)$$

wherein $R_f$ is perfluoroalkyl of 3 to 18 carbon atoms, $R_1$ is hydrogen or lower alkyl, m is 1 to 3 and n is 1 or 2.

2. A compound according to claim 1, wherein $R_f$ is perfluoroalkyl of 4 to 16 carbon atoms.

3. A compound according to claim 2, wherein $R_f$ is perfluoroalkyl of 4 to 12 carbon atoms.

4. A compound according to claim 1, wherein $R_1$ is hydrogen or methyl.

5. A compound according to claim 4, wherein $R_1$ is hydrogen.

6. A compound according to claim 1, wherein m is 1.

7. A compound according to claim 1, wherein n is 2.

8. A process for the manufacture of the compounds of the formula (1), which comprises

(a) reacting the compounds of the formula

$$R_f\!\left(CH_2\underset{\displaystyle R_1}{\underset{|}{C}H}\right)_m-S-CH_2CH_2X, \qquad (3)$$

wherein $R_f$, $R_1$ and m are as defined in claim 1 and X is a leaving group, with an organic or inorganic peroxide in a solvent at a temperature between 10 to 100°C to yield the compounds of the formula

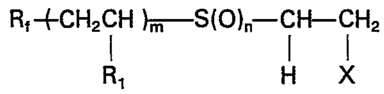
(2)

wherein $R_f$, $R_1$, m and n are as defined in claim 1 and X is as defined above,

(b) isolating the compounds of the formula (2),

(c) eliminating HX in the compounds of the formula (2) in the presence of an inert solvent or solvent mixture at a temperature between 0 and 100°C optionally in the presence of an acid acceptor, and

(d) isolating the compounds of the formula (1).

9. A process according to claim 8, wherein X is halogen, hydroxyl, lower alkoxy, lower acyloxy or alkalimetal sulfate.

10. The compounds of the formula

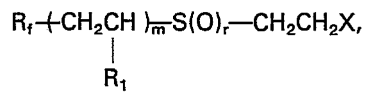
(2a)

wherein $R_f$, $R_1$, m and X are as defined in claim 8 and r is 0, 1 or 2.

11. Use of the compounds according to claim 10 for the manufacture of the compounds according to claim 1.

12. A polymer of the formula

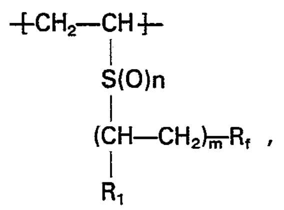
(7)

wherein $R_f$, $R_1$, m and n are as defined in claim 1.

13. A copolymer, which contains structure units of the formula (7) according to claim 12 and structure units of vinyl, alkene and acrylate comonomers.

14. Use of the polymer according to claim 12 and copolymers according to claim 13 to render oleophobic and hydrophobic cellulose and natural and synthetic polyamide materials.

15. A process for the preparation of the polymers according to claim 12, which comprises polymerizing the compounds of the formula

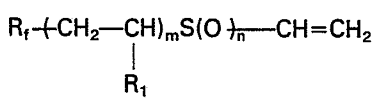
(1)

wherein $R_f$, $R_1$, m and n are as defined in claim 1, in the presence of an organic solvent and of actinic light or a free radical forming catalyst.

16. A process for the preparation of the copolymers according to claim 13, which comprises polymerizing the compounds of the formula

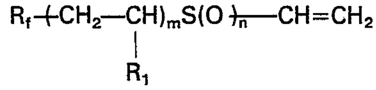
(1)

wherein $R_f$, $R_1$, m and n are as defined in claim 1, in the presence of an organic solvent, actinic light or a free radical forming catalyst and vinyl, alkene or acrylate comonomers.

17. A process for rendering oleophobic and hydrophobic cellulose and natural and synthetic polyamide materials, which comprises coating a polymer or copolymer according to claims 12 or 13 onto said substrates.

18. A process for rendering oleophobic and hydrophobic cellulose and natural and synthetic polyamide materials, which comprises applying a solution or emulsion of the compounds of the formula (1) to said substrates and polymerizing these compounds on the substrate optionally in the presence of an alkaline catalyst and a free radical catalyst.

**Patentansprüche**

1. Verbindungen der Formel (1)

$$R_f\!\left(CH_2-\underset{\displaystyle R_1}{\underset{|}{CH}}\right)_m S(O)_n-CH=CH_2, \qquad (1)$$

worin $R_f$ Perfluoralkyl mit 3 bis 18 C-Atomen und $R_1$ ein Wasserstoffatom oder Niedrigalkyl bedeuten, m 1 bis 3 und n 1 oder 2 darstellen.

2. Verbindungen gemäss Anspruch 1, worin $R_f$ Perfluoralkyl mit 4 bis 16 C-Atomen bedeutet.

3. Verbindungen gemäss Anspruch 2, worin $R_f$ Perfluoralkyl mit 4 bis 12 C-Atomen ist.

4. Verbindungen gemäss Anspruch 1, worin $R_1$ ein Wasserstoffatom oder Methyl ist.

5. Verbindungen gemäss Anspruch 4, worin $R_1$ ein Wasserstoffatom ist.

6. Verbindungen gemäss Anspruch 1, worin m 1 ist.

7. Verbindungen gemäss Anspruch 1, worin n 2 ist.

8. Verfahren zur Herstellung von Verbindungen der Formel (1), dadurch gekennzeichnet, dass man

(a) eine Verbindung der Formel (3),

$$R_f\!\left(CH_2\underset{\displaystyle R_1}{\underset{|}{CH}}\right)_m-S-CH_2CH_2X, \qquad (3)$$

worin $R_f$, $R_1$ und m die in Anspruch 1 angegebene Bedeutung haben und X eine Abgangsgruppe ist, mit einem organischen oder anorganischen Peroxid in Gegenwart eines Lösungsmittels und bei Temperaturen zwischen 10 und 100°C zu einer Verbindung der Formel (2) umsetzt,

$$R_f\!\left(CH_2\underset{\displaystyle R_1}{\underset{|}{CH}}\right)_m-S(O)_n-\underset{\displaystyle H}{\underset{|}{CH}}-\underset{\displaystyle X}{\underset{|}{CH_2}}, \qquad (2)$$

worin $R_f$, $R_1$, m und n die in Anspruch 1 angegebene Bedeutung haben und X wie zuvor definiert ist,

(b) die Verbindung der Formel (2) isoliert,

(c) aus den Verbindungen der Formel (2) in Gegenwart eines inerten Lösungsmittels oder Lösungsmittelgemisches bei Temperaturen zwischen 0 und 100°C und gegebenenfalls in Gegenwart eines Säureakzeptors HX eliminiert, und

(d) die Verbindung der Formel (1) isoliert.

9. Verfahran gemäss Anspruch 8, worin X Halogen, Hydroxyl, Niederalkoxy, Niederacyloxy oder Alkalimetallsulfat ist.

10. Verbindungen der Formel (2a),

$$R_f\!\left(CH_2\underset{\displaystyle R_1}{\underset{|}{CH}}\right)_m S(O)_r-CH_2CH_2X, \qquad (2a)$$

worin $R_f$, $R_1$, m und X die in Anspruch 8 angegebene Bedeutung haben und r 0, 1 oder 2 ist.

11. Verwendung von Verbindungen gemäss Anspurch 10 zur Herstellung von Verbindungen der Formel (1) gemäss Anspruch 1.

12. Polymere der Formel (7)

$$\begin{array}{l} \left(CH_2-CH\right) \\ \qquad\;\; | \\ \qquad S(O)n \\ \qquad\;\; | \\ \qquad (CH-CH_2)_m R_f\,, \\ \qquad\;\; | \\ \qquad R_1 \end{array} \qquad (7)$$

worin $R_f$, $R_1$, m und n die in Anspruch 1 angegebenen Bedeutung haben.

13. Copolymere, welche Strukturelemente der Formel (7) gemäss Anspruch 12 und Strukturelemente von Vinyl-, Alken- und Acrylat-comonomeren enthalten.

14. Verwendung von Polymeren und Copolymeren gemäss den Ansprüchen 12 und 13 zur öl- und wasserabstossenden Ausrüstung von Cellulose und natürlichen und synthetischen Polyamidmaterialien.

15. Verfahren zur Herstellung von Polymeren gemäss Anspruch 12, dadurch gekennzeichnet, dass man eine Verbindung der Formel (1)

$$R_f\text{-}(CH_2-\underset{\displaystyle R_1}{\underset{|}{CH}})_mS(O)_n-CH=CH_2\ , \qquad (1)$$

worin $R_f$, $R_1$, m und n die in Anspruch 1 angegebene Bedeutung haben, in Gegenwart eines organischen Lösungsmittels unter Einwirkung von aktinischem Licht oder in Gegenwart eines freie Radikale bildenden Katalysators polymerisiert.

16. Verfahren zur Herstellung von Copolymeren gemäss Anspruch 13, dadurch gekennzeichnet, dass man eine Verbindung der Formel (1)

$$R_f\text{-}(CH_2-\underset{\displaystyle R_1}{\underset{|}{CH}})_mS(O)_n-CH=CH_2\ , \qquad (1)$$

worin $R_f$, $R_1$, m und n die in Anspruch 1 angegebene Bedeutung haben, und ein Vinyl-, Alken- oder Acrylatcomonomer in Gegenwart eines Lösungsmittels unter Einwirkung von Licht oder in Gegenwart eines freie Radikale bildenden Katalysators polymerisiert.

17. Verfahren zur öl- und wasserabstossenden Ausrüstung von Cellulose und natürlichen und synthetischen Polyamidmaterialien, bei dem besagte Substrate mit einem Polymer oder Copolymer gemäss den Ansprüchen 12 oder 13 beschichtet wird.

18. Verfahren zur öl- und wasserabstossenden Ausrüstung von Cellulose und natürlichen und synthetischen Polyamidmaterialien, bei dem besagte Substrate mit einer Lösung oder Emulsion von Verbindungen der Formel (1) behandelt werden, und diese Verbindungen gegebenenfalls in Gegenwart eines alkalischen und eines freie Radikale bildenden Katalysators polymerisiert.

**Revendications**

1. Composé répondant à la formule

$$R_f\text{-}(CH_2-\underset{\displaystyle R_1}{\underset{|}{CH}})_mS(O)_n-CH=CH_2 \qquad (1)$$

dans laquelle $R_f$ est un groupe perfluoroalkyle en $C_3$ à $C_{18}$, $R_1$ est l'hydrogène ou un alkyle inférieur, m est 1 à 3 et n est 1 ou 2.

2. Composé suivant la revendication 1, dans lequel $R_f$ est un groupe perfluoroalkyle en $C_4$ à $C_{16}$.
3. Composé suivant la revendication 2, dans lequel $R_f$ est un groupe perfluoroalkyle en $C_4$ à $C_{12}$.
4. Composé suivant la revendication 1, dans lequel $R_1$ est un hydrogène ou un groupe méthyle.
5. Composé suivant la revendication 4, dans lequel $R_1$ est l'hydrogène.
6. Composé suivant la revendication 1, dans lequel m est 1.
7. Composé suivant la revendication 1, dans lequel n est 2.
8. Procédé de préparation des composés répondant à la formule (1), qui consiste

(a) à faire réagir les composés répondant à la formule

$$R_f\text{-}(CH_2\underset{\displaystyle R_1}{\underset{|}{CH}})_m-S-CH_2CH_2X \qquad (3)$$

dans laquelle $R_f$, $R_1$ et m sont tels que définis dans la revendication 1, et X est un groupe partant, avec un peroxyde organique ou minéral dans un solvant, à une température entre 10 et 100°C, pour donner les composés répondant à la formule

$$R_f\text{-}(CH_2\underset{\displaystyle R_1}{\underset{|}{CH}})_m-S(O)_n-\underset{\displaystyle H}{\underset{|}{CH}}-\underset{\displaystyle X}{\underset{|}{CH_2}} \qquad (2)$$

dans laquelle $R_f$, $R_1$, m et n sont tels que définis dans la revendication 1, et X est tel que défini ci-dessus,

(b) à isoler les composés répondant à la formule (2),

(c) à éliminer HX dans les composés répondant à la formule (2) en présence d'un solvant inerte ou d'un mélange de solvants, à une température entre 0 et 100°C, en présence si on le désire d'un accepteur d'acide, et

(d) à isoler les composés répondant à la formule (1).

9. Procédé suivant la revendication 8, dans lequel X est un halogène, un groupe hydroxyle, alcoxy inférieur, acyloxy inférieur ou sulfate de métal alcalin.

10. Composés répondant à la formule

$$R_f\text{-}\!\left(CH_2\underset{\underset{\displaystyle R_1}{|}}{C}H\right)_{\!m}\text{-}S(O)_r\text{—}CH_2CH_2X \qquad (2a)$$

dans laquelle $R_f$, $R_1$, m et X sont tels que définis dans la revendication 8 et r est 0, 1 ou 2.

11. Utilisation des composés suivant la revendication 10, pour la préparation des composés suivant la revendication 1.

12. Polymère répondant à la formule

$$\left(CH_2\text{—}\underset{\underset{\displaystyle S(O)n}{|}}{C}H\right) \qquad (7)$$
$$\text{with } S(O)n \text{—} \left(\underset{\underset{\displaystyle R_1}{|}}{C}H\text{—}CH_2\right)_{m}\text{-}R_f$$

dans laquelle $R_f$, $R_1$, m et n sont tels que définis dans la revendication 1.

13. Copolymère qui contient des motifs répondant à la formule (7) suivant la revendication 12 et des motifs de comonomères vinyle, alcène et acrylate.

14. Utilisation du polymère suivant la revendication 12 et de copolymères suivant la revendication 13 pour rendre oléophobes et hydrophobes des matières de cellulose et de polyamide naturels et synthétiques.

15. Procédé pour la préparation des polymères suivant la revendication 12, qui consiste à polymériser les composés répondant à la formule

$$R_f\text{-}\!\left(CH_2\text{—}\underset{\underset{\displaystyle R_1}{|}}{C}H\right)_{\!m}S(O)_{n}\text{—}CH{=}CH_2 \qquad (1)$$

dans laquelle $R_f$, $R_1$, m et n sont tels que définis dans la revendication 1, en présence d'un solvant organique et de lumière actinique ou d'un catalyseur radicalaire.

16. Procédé pour la préparation des copolymères suivant la revendication 13, qui consiste à polymériser les composés répondant à la formule

$$R_f\text{-}\!\left(CH_2\text{—}\underset{\underset{\displaystyle R_1}{|}}{C}H\right)_{\!m}S(O)_{n}\text{—}CH{=}CH_2 \qquad (1)$$

dans laquelle $R_f$, $R_1$, m et n sont tels que définis dans la revendication 1, en présence d'un solvant organique, de lumière actinique ou d'un catalyseur radicalaire et de comonomères vinyle, alcène ou acrylate.

17. Procédé pour rendre oléophobes et hydrophobes des matières cellulosiques et de polyamides naturels et synthétiques, qui consiste à appliquer un polymère ou copolymère suivant les revendications 12 ou 13 sur ces substrats.

18. Procédé pour rendre oléophobes et hydrophobes des matières cellulosiques et de polyamides naturels et synthétiques, qui consiste à appliquer une solution ou une émulsion des composés répondant à la formule (1) sur ces substrats et à polymériser ces composés sur le substrat, en présence si on le désire d'un catalyseur alcalin et d'un catalyseur radicalaire.